# EUROPÄISCHE PATENTSCHRIFT

(11) **EP 1 714 606 B1**
(45) Veröffentlichungstag und Bekanntmachung des Hinweises auf die Patenterteilung: **03.09.2008**
(21) Anmeldenummer: 06006026.6
(22) Anmeldetag: 23.03.2006
(51) Int. Cl.: A61B 1/005, A61B 1/07, A61B 1/00

(54) **Endoskop**
Endoscope
Endoscope

(30) Priorität: 22.04.2005 DE 102005018825
(43) Veröffentlichungstag der Anmeldung: 25.10.2006
(73) Patentinhaber: Polydiagnost GmbH, 85276 Pfaffenhofen (DE)
(72) Erfinder: Schaaf, Hansgeorg, 85293 Reichertshausen (DE)
(74) Vertreter: Eisenführ, Speiser & Partner

(56) Entgegenhaltungen:
- WO-A-20/04105594
- DE-A1- 19 956 516
- US-A- 4 772 093
- US-A- 5 569 161

## Beschreibung

Die Erfindung betrifft ein Endoskop mit einer flexiblen und mehrere Lumen aufweisenden Kathetersonde nach dem Oberbegriff des Patentanspruches 1.

Ein derartiges aus US 5,569,161 bekanntes Endoskop beinhaltet in der Kathetersonde ein Optiklumen, in welchem ein Optikstrang mit in Katheterlängsrichtung verlaufenden Lichtleitern vorgesehen ist. Die distale Öffnung des Optiklumens wird von einer lichtdurchlässigen Abdeckung verschlossen. Zwischen dem proximalen Optiklumenende und dem proximalen Optikstrangende ist eine federnd wirkende Längenausgleichseinrichtung vorgesehen, mit welcher das distale Ende des Optikstranges gegen die lichtdurchlässige Abdeckung in Anlage gehalten wird. Bei einem derartigen Endoskop ist es nicht erforderlich, zwischen einzelnen Anwendungen den Optikstrang zu sterilisieren, da dieser während der Patientenbehandlung hermetisch von den Komponenten des Endoskops, insbesondere durch die distale Abdeckung am Optiklumen und die Kathetersonde gegenüber dem Körperinnern, abgetrennt ist.

Bei Endoskopen, wie sie beispielsweise aus DE 100 45 036 C1 bekannt sind, kann das distale Sondenende mit Hilfe eines Steuerelementes, beispielsweise Zugdrahtes oder Zugseils abgewinkelt und gegebenenfalls um 180° gegenüber der Kathetersondenachse zurückgebogen werden. Dabei und auch bei der Bewegung aus der gebogenen Position zurück in die in Längsrichtung ausgerichtete Position des distalen Sondenendes treten auch bei Anwendung einer Längenausgleichseinrichtung relativ hohe Änderungen der Kraft, mit welcher das distale Ende des Optikstranges an der Abdeckung anliegt, auf. Ferner, kann das distale Ende des Optikstranges von der Abdeckung auch wegbewegt werden. Hieraus resultiert eine Einengung des Blickwinkels und damit des Sichtbereiches, welcher über den Optikstrang beobachtet wird. Andererseits können die auf die Abdeckung ausgeübten Anlagekräften so groß werden, dass die Gefahr des Lösens der Abdeckung vom distalen Optiklumenende besteht.

Aufgabe der Erfindung ist es, ein Endoskop der eingangs genannten Art zu schaffen, bei dem in allen in der Praxis vorkommenden Betriebszuständen des Endoskops, insbesondere der Kathetersonde eine einwandfreie Anlage des distalen Ende des Optikstranges an der distalen Lumenabdeckung gewährleistet ist.

Diese Aufgabe wird erfindungsgemäß durch die Merkmale des Patentanspruches 1 gelöst.

Bei der Erfindung beinhaltet die Längenausgleichseinrichtung einen Kraftspeicher bevorzugt in Form einer Feder, insbesondere Druckfeder. Die Feder greift an zwei Krafteinleitungsstellen an, von denen die eine Krafteinleitungsstelle starr mit dem proximalen Optikstrangende und die andere Krafteinleitungsstelle starr mit dem proximalen Optiklumenende verbunden ist. im normalen Endoskopbetrieb werden in die Krafteinleitungsstelle entgegengesetzt wirkende Kräfte eingeleitet, welche in einer bestimmten Anlagekraft resultieren, mit der das distale Ende des Optikstranges auf die lichtdurchlässige Abdeckung des Optiklumens zur Anlage gebracht wird. Um Beschädigungen an der Abdeckung und am distalen Ende des Optikstranges zu verhindern, ist die Kraft auf niedrige Werte, beispielsweise in der Größenordnung von 2 N zu begrenzen. Der zu erzielende Längenausgleich beim Abbiegen der Kathetersonde, welcher sich in einer Änderung des Abstandes zwischen den beiden Krafteinleitungsstellen auswirkt, ist daher unter Beibehaltung der erforderlichen Anlage des distalen Endes des Optikstranges an der Abdeckung nur in engen Grenzen möglich. Damit sind die Bewegungsmöglichkeiten, insbesondere der Grad der Abbiegung im distalen Bereich der Kathetersonde begrenzt.

Bei der Erfindung wird der Umfang der Bewegungsmöglichkeit, insbesondere der Grad der möglichen Abbiegung des distalen Kathetersondenendes zu bis 180° gegenüber der Sondenachse und darüber hinaus erweitert, wobei eine einwandfreie Anlage des distalen Endes des Optikstranges an der lichtdurchlässigen Abdeckung des Optiklumens gewährleistet bleibt.

Hierzu kann der Abstand der mit dem proximalen Optikstrangende verbundenen Krafteinleitungsstelle der Längenausgleichseinrichtung vom proximalen Optikstrangende verändert werden. Eine weitere Möglichkeit besteht darin, dass der Abstand der starr mit dem proximalen Optiklumenende verbundenen Krafteinleitungsstelle der Längenausgleichseinrichtung gegenüber dem proximalen Optiklumen verstellt werden kann. Es können auch beide Alternativen gleichzeitig vorgesehen sein.

Die Vorrichtung zur Abstandseinstellung besteht vorzugsweise aus einer Kolbenzylinderanordnung. Bei einer Alternative sind der Zylinder mit dem proximalen Optiklumenende und der Kolben mit dem proximalen Optikstrangende starr verbunden. Bei einer anderen Alternative sind der Kolben mit dem proximalen Optiklumenende und der Zylinder mit dem proximalen Optikstrangende starr verbunden. Zur Erzielung der Abstandseinstellung ist der Kolben im Zylinder in unterschiedlichen axialen Positionen fixierbar. Zum Fixieren kann vorzugsweise eine Klemmeinrichtung, beispielsweise eine in die Zylinderwand mit Gewindeeingriff eingesetzte Klemmschraube vorgesehen sein, mit welcher der Kolben in der gewünschten axialen Position im Zylinder festgehalten wird. Auf diese Weise ist eine stufenlose Abstandseinstellung möglich. Natürlich kann auch eine gestufte Abstandseinstellung vorgesehen sein, beispielsweise dadurch, dass am Kolben oder einer Kolbenstange in bestimmten axialen Abständen Klemmstellen bzw. Fixierstellen vorgesehen sind.

Die Kolbenzylinderanordnung, mit welcher die Abstandseinstellung durchgeführt wird und die Längenausgleichseinrichtung sind vorzugsweise in Reihe (hintereinander) zwischen dem proximalen Optikstrangende und dem proximalen Optiklumenende angeordnet. Auf diese Weise erreicht man eine optimale Abstandseinstellung in axialer Richtung des Optikstranges und der Kathetersonde.

Diese Abstandseinstellung kann insbesondere dann durchgeführt werden, wenn die Längenausgleichsbewegung der Längenausgleichseinrichtung und die damit voreingestellte Anlagekraft des .distalen Optikstrangendes an der Abdeckung in den Grenzbereich kommt. Auch eine Verringerung des Blickwinkels, welche aus einem Lösen des distalen Optikstrangendes von der Abdeckung resultiert, kann durch die Abstandsveränderung kompensiert bzw. rückgängig gemacht werden.

Zur Verbesserung der mechanischen Festigkeit der lichtdurchlässigen Abdeckung und zur Verbesserung der Positionierung der lichtdurchlässigen Abdeckung im distalen Optiklumenende kann eine in das distale Optiklumenende eingeklebte oder fest verankerte Fassung, welche mit einer zylindrischen Mantelfläche an der Innenseite des distalen Optiklumenende befestigt ist, zur Aufnahme und Positionierung der lichtdurchlässigen Abdeckung vorgesehen sein. Um eine einwandfreie Verankerung und Befestigung der Abdeckung zu erreichen, ist die Mantelfläche, welche insbesondere kreiszylindrisch ausgebildet ist, mit einer oder mehreren Vertiefungen ausgestattet, die mit einem Klebemittel angefüllt sind. Als Klebemittel eignet sich ein härtbarer Kleber, der in flüssiger Form in die Vertiefungen und zwischen die zylindrische Mantelfläche und die Innenseite des Optiklumens eingebracht wird und anschließend ausgehärtet wird. Das Klebemittel ist vorzugsweise eine Verbindung auf Epoxidharzbasis. Eine derart ausgebildete Fassung für die Abdeckung ist geeignet, die mechanische Stabilität der Abdeckung und der Fixierung der Abdeckung im distalen Optiklumenende zu gewährleisten.

Hierdurch erreicht man eine Verbesserung der mechanischen Stabilität der distalen Abdeckung im Optiklumen und ihrer Anordnung und Halterung. Somit können unter Umständen auftretende Kraftspitzen der Anlagekraft, mit welcher das distale Ende des Optikstranges an der Abdeckung anliegt, wirksam aufgenommen werden.

In vorteilhafter Weise ist die Fassung hülsenförmig mit Kreiszylinderform ausgebildet. Am distalen Ende kann die Hülse einen zur Hülsenachse hin gerichteten einwärts gebogenen Hülsenrand aufweisen. An diesem Hülsenrand, der eine innenliegende Anlageschulter bildet, liegt die Abdeckung an. Es ist auch möglich, am distalen Hülsenende eine innenliegende Anlageschulter einzuformen, an welcher die Abdeckung nach außen hin abgestützt anliegt. Die Fassung besteht vorzugsweise aus Metall, insbesondere Edelstahl.

Zum Einfüllen des Klebemittels kann im Bereich der axialen Ausdehnung der Fassung im Material des Optiklumens eine von außen bis zur Mantelfläche der Fassung ragende Öffnung vorgesehen sein. Durch diese Öffnung kann das Klebemittel in flüssiger Form zwischen die Mantelfläche und die Innenseite des Optiklumens sowie in die Vertiefungen der Mantelfläche eingebracht werden und anschließend ausgehärtet werden.

In der Fassung kann wenigstens ein optisch wirksames Bauteil fest oder auswechselbar angeordnet werden. Das optisch wirksame Bauteil kann eine oder mehrere optische Linsen aufweisen oder auch aus einem Videochip bestehen oder enthalten, dessen Verbindungsdrähte durch das Optiklumen geführt sind. Für die Positionierung des Videochips können die Verbindungsdrähte entsprechend ausgebildet sein oder der Videochip kann mit Hilfe eines drahtförmigen und spiralförmigen Schubelements in die distale Position gebracht werden. Die Signale des Videochips können auch mit Transpondertechnik drahtlos übertragen werden Es genügen dann Lichtleiter zum Ausleuchten des zu beobachtenden Raums.

Ferner .kann der Optikstrang oder ein zusätzlicher Lichtleiterstrang in der Weise ausgebildet sein, dass mit ihm eine Anregungsstrahlung oder Anregungslicht, mit welcher bzw. mit welchem Autofluoreszenz in biologischen Zellen oder Geweben, insbesondere bei der Krebsfrüherkennung übertragen werden kann. Entsprechende Anregungslichtquellen und Auswerteeinrichfungen, welche an das Endoskop angeschlossen sind; sind beispielsweise aus DE 198 00 312 A1 und 101 16 859 A1 bekannt.

In vorteilhafter Weise können für Kathetersonden mit unterschiedlichen Längen ein Optikstrang oder Optikstränge mit einheitlicher Länge vorgesehen sein. Um den Längenausgleich bei den unterschiedlichen Kathetersonden zu erreichen, können bei einem Ausführungsbeispiel der Erfindung Schutzhüllen mit unterschiedlichen Längen vorgesehen sein. Durch diese Schutzhüllen werden die über das proximale Ende der jeweiligen Kathetersonde überstehenden Teile des Optikstranges geführt. Auf diese Weise ist es möglich, einen Optikstrang oder Optikstränge mit einheitlicher Länge für unterschiedliche Einsatzzwecke des Endoskops zu verwenden. Beispielsweise können Kathetersondenlängen mit 20 cm, 30 cm, 60cm, 85 cm und 185 cm Länge zum Einsatz kommen. Für kürzere Längen der Kathetersonden kann ein Optikstrang mit verkürzter Länge zum Einsatz kommen. Beispielsweise können Optikstränge mit 150 cm Länge und 300 cm Länge für die verschiedenen Kathetersondenlängen in Bereitschaft gehalten werden.

In bevorzugter Weise werden die Optikstränge in schlauchförmigen Dispensern außerhalb der Kathetersonde in Bereitschaft gehalten werden, wobei der Dispenser an seinem Ende einen Anschluss für eine Spüleinrichtung aufweist, so dass eine einwandfreie Reinigung und Sterilisation des Optikstranges im Dispenser möglich ist.

Bevorzugt kommt die Erfindung zum Einsatz bei einem Endoskop mit einer Kathetersonde, deren distales Ende mit Hilfe eines Steuerelementes in verschiedenen Richtungen gegebenenfalls bis 180° und darüber abgebogen werden kann. Dieses Steuerelement erstreckt sich entlang der Kathetersonde vorzugsweise in einem Steuerlumen der Kathetersonde und ist mit dem distalen Sondenende oder in der Nähe davon befestigt und wird am proximalen Ende betätigt. Ein derartiges Endoskop ist beispielsweise aus DE 100 45 036 C1 bekannt.

Anhand der Figuren wird an Ausführungsbeispielen die Erfindung noch näher erläutert.

Es zeigt
- Fig. 1: ein Ausführungsbeispiel der Erfindung in schematischer Darstellung;
- Fig. 2: eine schnittbildliche Darstellung durch eine Längenausgleichseinrichtung, welche beim Ausführungsbeispiel der Figur 1 zur Anwendung kommt;
- Fig. 3: eine schnittbildliche Darstellung des distalen Endes der Kathetersonde im Bereich des Optiklumens;
- Fig. 4: in perspektivischer Darstellung eine hülsenförmige Aufnahme für eine das distale Ende des Optiklumens verschließende lichtdurchlässige Abdeckung;und
ein Ausführungsbeispiel für einen Dispenser, in welchem ein bei der Erfindung zu Anwendung kommender Optikstrang steril in Bereitschaft gehalten werden kann.

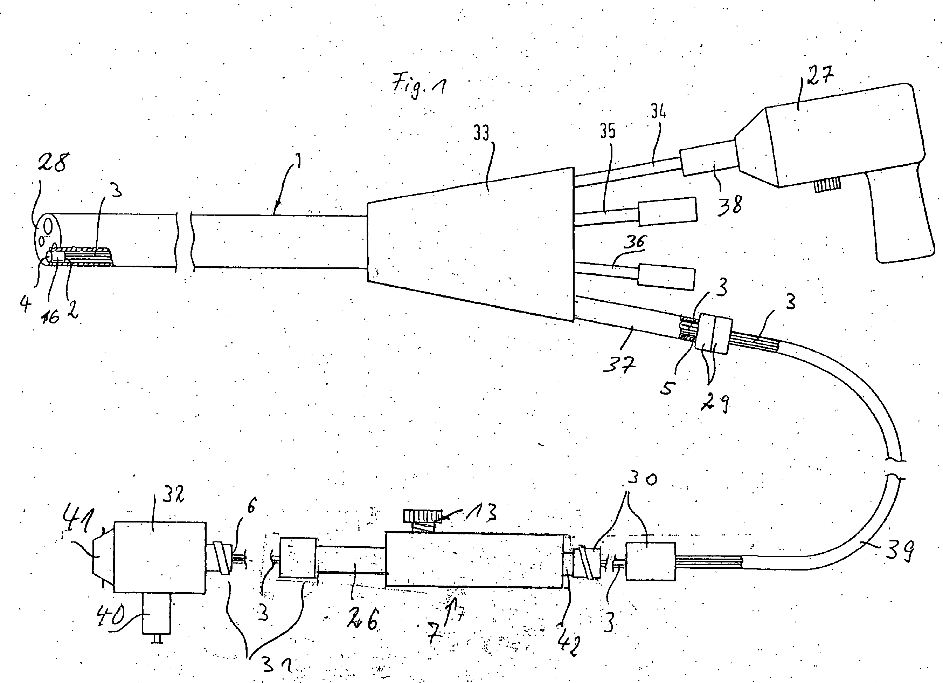

Das in Figur 1 dargestellte Ausführungsbeispiel eines Endoskops besitzt einen Handgriff 27, der auch eine andere bekannte Ausführungsform haben kann, und eine daran lösbar zu befestigende Kathetersonde 1. Die Sonde 1 ist als mehrlumige Sonde ausgebildet und kann beispielsweise ein Arbeitslumen oder mehrere Arbeislumen für chirurgische Werkzeuge und wenigstens ein Optiklumen 2 für einen Optikstrang 3 aufweisen. Für den eine Beleuchtungsoptik und Beobachtungsoptik aufweisenden Optikstrang 3 können auch getrennte Optiklumina vorgesehen sein. Desgleichen können zum Spülen und Absaugen separate Lumina in der Sonde 1 vorgesehen sein.

Ferner kann die Kathetersonde 1 ein Steuerelement, beispielsweise in Form eines Zugseiles oder Zugdrahtes aufweisen. Das längliche Steuerelement ist, wie beispielsweise aus DE 100 45 036 C1 bekannt ist, mit einem distalen Sondenende 28 fest verbunden oder in der Nähe davon befestigt und erstreckt sich in axialer Richtung entlang der Sonde und ist an dieser beweglich geführt. Durch das Steuerelement kann das distale Endstück der Kathetersonde 1 gebogen werden.

Das mittels des Steuerelementes abbiegbare distale Sondenende 28 kann in der Weise ausgebildet sein, wie es aus DE 201 18 886 U oder aus DE 199 28 272 A1 bekannt ist.

Die Kathetersonde 1 besteht aus einem biegbaren Material, insbesondere biokompatiblen Kunststoff. Vorzugsweise ist sie als Einmalteil ausgebildet; welches nach einem chirurgischen Einsatz vom Handgriff 27 gelöst und entsorgt wird. Bei einem erneuten chirurgischen Eingriff wird am Handgriff 27 eine neue Kathetersonde befestigt, welche als Einmalteil steril in Bereitschaft gehalten wird.

Am proximalen Ende besitzt die Kathetersonde 1 ein Sondenansatzstück 33 aus einem starren, festen Material. Dieses Material kann ebenfalls ein Kunststoffmaterial sein. Am Sondenansatzstück 33 befinden sich proximale Lumenausgänge 34, 35, 36 und 37. Durch den Lumenausgang 34 ist das Steuerelement geführt. Der Lumenausgang 35 ist beispielsweise einem Ballonlumen, der Lumenausgang 46 ist beispielsweise einem Arbeitslumen und der Lumenausgang 37 ist beispielsweise dem Optiklumen 2 zugeordnet. In bekannter Weise sind die Ausgänge zur Bildung von Verbindungseinrichtungen mit Kupptungseiementen ausgestattet, beispielsweise für einen Bajonettverschluss, Luer-Lock oder mit ähnlichen Kupplungs- und Anschlussstücken.

Die Kathetersonde 1 kann über den starren, röhrchenförmigen Lumenausgang 34 für das Steuerelement 13 drehfeste mit einem Drehteil eines Drehlagers 38 am Handgriff 27, insbesondere am Handgriffgehäuse verbunden sein. Die Kathetersonde 1 kann auch drehfest mit dem Handgriff 27 oder dem Sondenansatzstück 33 verbunden sein.

Durch den Lumenausgang 37 kann der Optikstrang 3, welcher eine Beleuchtungs- und eine Beobachtungsoptik beinhaltet, in das Optiklumen 2 der Kathetersonde 1 eingesetzt werden. Für die beiden Stränge der Beleuchtungsoptik und der Beobachtungsoptik können auch separate Optiklumina vorgesehen sein. Das jeweilige distale Ende des Optiklumens 5 ist mit einer lichtdurchlässigen Abdeckung 4 hermetisch abgeschlossen. Auf diese Weise wird eine Kontamination des Optikstranges 3 am Zielort verhindert. Der übrige Teil des Optikstranges 3 ist durch die von der Kathetersonde 1 gebildete Ummantelung gegen Kontamination geschützt. Der Optikstrang 3 wird, wie noch erläutert wird, mit Hilfe einer Längenausgleichseinrichtung 7 bis zur lichtdurchlässigen Abdeckung 4 im Optiklumen 2 nach vorne verschoben und mit einem geringen Anlagedruck, z. B. etwa 2 N zur Anlage gebracht.

Der über das proximale Ende der Kathetersonde 1 und den Lumenausgang 37 überstehende Optikstrang 3 wird in axialer Richtung in einem flexiblen schlauchförmigen Schutzhülle 39 geführt. Die Schutzhülle 39 bildet einen Schutz gegen äußere Einflüsse. Die Schutzhülle 39 besitzt am vorderen (distalen) Ende ein Kupplungsstück, welches mit dem proximalen Lumenausgang 37 eine lösbare Verbindungseinrichtung 29, beispielsweise Luer-Lock, bildet. Das hintere (proximale) Hüllenende ist ebenfalls mit einem Kupplungselement ausgestattet, welches lösbar mit einem Anschlussstück mit der Längenausgleichseinrichtung 7 unter Bildung einer lösbaren Verbindungseinrichtung 30 verbunden werden kann. Die lösbare Verbindungseinrichtung 30 kann als Luer-Lock oder dergleichen ausgebildet sein.

Der Optikstrang 3 wird ferner durch eine noch zu erläuternde Längenausgleichseinrichtung 7 geführt und an seinem proximalen Optikstrangende 6 an einem Adapter 32 befestigt. Über einen Beleuchtungsanschluss 40 des Adapters 32 kann der entsprechende Lichtleiterteil des Optikstranges 3 an eine Beleuchtungslichtquelle angeschlossen werden. Ferner kann an den Adapter 32 ein Ocular- und/oder Kamerasystem zur Erfassung der vom Optikstrang 3 übermittelten Bildinformationen angeschlossen werden. Hierzu dient ein Ocularanschluss 41 am Adapter 32.

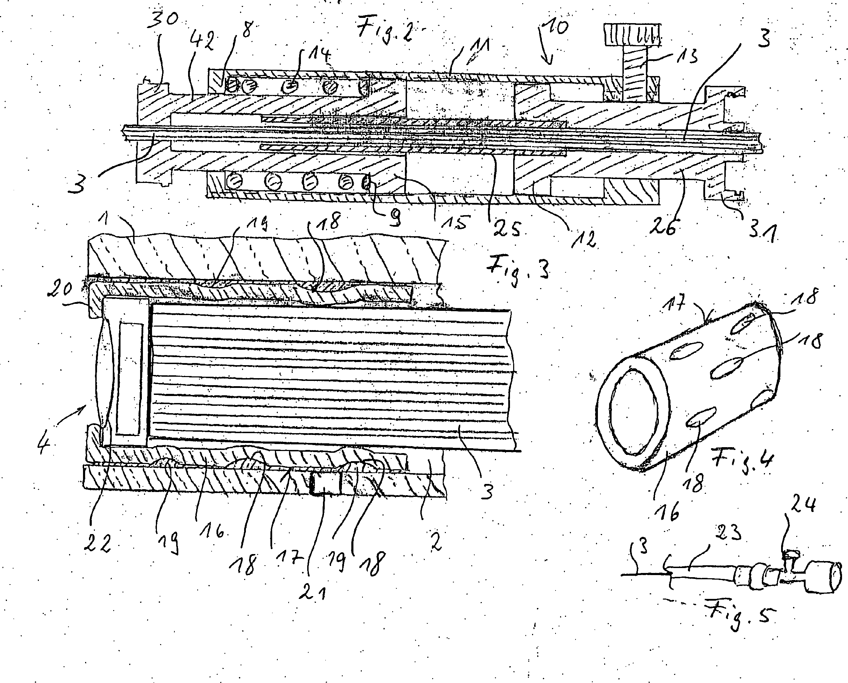

Die in Figur 2 dargestellte Ausführungsform der Längenausgleichseinrichtung 7 besitzt einen Zylinder 11, welcher vorzugsweise als Gaszylinder ausgebildet ist. Im Zylinder ist ein Kolben 15 vorgesehen, welcher fest oder einstückig mit dem Kupplungsteil der Verbindungseinrichtung 30 über eine Kolbenstange 42 verbunden ist. Über die Schutzhülle 39, welche in ihrer Längsrichtung starr ausgebildet ist, wird auf diese Weise der Kolben 15 starr mit dem Optiklumenende 5, welches an der Verbindungseinrichtung 29 befestigt ist, verbunden. Das Optiklumenende 5 kann auch zur starren Verbindung mit dem Kolben 15 am Sondenansatzstück 33 vorgesehen sein.

Am in der Figur 2 linken Ende des Zylinders 11 wird eine Abstützstelle durch eine mit dem Zylinder 11 fest verbundene gelochte Endscheibe, welche auch aus einem Stück mit dem Zylinder 11 bestehen kann, gebildet. Diese Abstützstelle bildet eine Krafteinleitungsstelle 8 für eine Feder 17, welche ferner am Kolben 15 in einer, Krafteinleitungsstelle 9 abgestützt ist. Die Krafteinleitungsstelle 9 ist wie schon erläutert, in axialer Richtung des Optikstranges 3, fest mit dem proximalen Optiklumenende 5 verbunden. Die andere Krafteinleitungsstelle 8, an welcher die als Druckfeder ausgebildete Feder 14 abgestützt ist, ist in axialer Richtung des Optiklumenstranges 3 starr und fest mit dem proximalen Optikstrangende 6 verbunden. Diese Verbindung erfolgt über den Zylinder 11, eine als Klemmeinrichtung 13 ausgebildete Fixiereinrichtung, mit welcher der Zylinder 11 mit einer Kolbenstange 26 verbunden ist. Die Kolbenstange 26 ist fest oder einstückig mit einem Kupplungsteil der Verbindungseinrichtung 31 verbunden. Wie aus Figur 1 zu ersehen ist, verbindet die Verbindungseinrichtung 31 die Kolbenstange 26 fest mit dem Adapter 32 und damit mit dem Optikstrangende 6.

Im Zylinder 11 ist ein weiterer, mit der Kolbenstange 26 verbundener Kolben 12 angeordnet, welcher beim Lösen der Klemmeinrichtung 13 in axialer Richtung beweglich geführt ist. Der Zylinder 11 und der verstellbare Kolben 12 bilden zusammen mit der lösbaren Fixiereinrichtung (Klemmeinrichtung 13) eine Abstandseinstelleinrichtung, mit welcher der Abstand der Krafteinleitungsstelle 8 vom Optikstrangende 6 verstellt werden kann. Hierzu kann die Klemmeinrichtung 13 in verschiedenen axialen Positionen an der Kolbenstange 26, welche fest oder einstückig mit dem Kolben 12 verbunden ist, fixiert werden. Gleichzeitig verschiebt sich dabei in axialer Richtung die Krafteinleitungsstelle 8 am Zylinder 11, wodurch ferner die jeweils vorhandene Druckkraft, welche von der Feder 14 zwischen den beiden Krafteinleitungsstellen 8 und 9 am Zylinder 11 und Kolben 15 ausgeübt wird, geändert wird. Die an den Krafteinwirkungsstellen 8 und 9 wirkenden Druckkräfte bewirken, dass das distale Ende des Optikstranges 3 mit einer bestimmten Anlagekraft an der lichtdurchlässigen Abdeckung 4 anliegt. Beim Abbiegen der Kathetersonde 1 während der Behandlung und insbesondere beim Abbiegen des distalen Endstückes der Kathetersonde 1 wird in bestimmtem Unfang durch die Druckfeder und die Verschiebbarkeit des Kolbens 15 im Zylinder 11 ein Längenausgleich zwischen der Kathetersonde 1 bzw. dem Optiklumen 2 und dem Optikstrang 3 erzielt. Innerhalb dieses Längenausgleiches ist gewährleistet, dass das distale Optikstrangende an der Abdeckung 4 anlegt. Hierzu können die Kolbenstange 42 und der Kolben 15 in der Figur 2 um einen bestimmten Betrag aus dem Zylinder 11 herausgezogen werden, so dass von der Feder 14 die gewünschte Druckkraft ausgeübt wird. Bei starken Abbiegungen oder auch bei der Führung der Kathetersonde 1 in einem äußeren Katheterrohr, kann es insbesondere bei großer Katheterlänge geschehen, dass der gewünschte Längenausgleich nicht mehr stattfindet und das distale Ende des Optikstranges 3 mit zu hoher Kraft auf die Abdeckung 4 gedrückt wird oder in Gegenrichtung das distale Ende des Optikstranges 3 sich von der Abdeckung 4 löst. Um dies zu vermeiden, ist die oben erläuterte Abstandseinstelleinrichtung, welche durch den zusätzlichen Kolben 12 im Zylinder 11 gebildet wird, vorgesehen. Diese Abstandseinstelleinrichtung wird somit ebenfalls von einer Kolbenzylinderanordnung 10 gebildet, welche in Form der Klemmeinrichtung 13 eine Fixiereinrichtung für die Fixierung des Kolbens 12 in verschiedenen axialen Positionen innerhalb des Zylinders 11 noch aufweist. Wie schon erläutert, ist der Kolben 12 starr über die Kolbenstange 26 und die Verbindungseinrichtung 31 mit dem proximalen Optikstrangende 6 verbunden.

Wie aus der Figur 2 zu ersehen ist, wird der Optikstrang 3 in axialer Richtung durch die Längenausgleichseinrichtung 7 so geführt, dass die Kolben 12 und 15 beweglich gegenüber dem Optikstrang 3 sind. Zur axialen Ausrichtung kann am Kolben 12 ein Führungsrohr 25 vorgesehen sein, welches verschiebbar in den Kolben 15 ragt. Es ist natürlich auch möglich, am Kolben 15 das Führungsrohr 25 zu befestigen und es im Kolben 12 verschiebbar zu lagern.

In gleicher Weise wirkt die Längenausgleichseinrichtung 7, wenn der Kolben 12 und seine Kolbenstange 26 starr mit dem Optiklumenende 5 verbunden ist und der Kolben 15 mit seiner Kolbenstange 42 starr mit dem Optikstrangende 6 verbunden ist. Hierzu ist es lediglich erforderlich, dass das mit der Kolbenstange 42 verbundene Kupplungselement an das Kupplungselement des Adapters 32 und das mit der Kolbenstange 26 verbundene Kupplungselement mit dem Kupplungselement am proximalen Ende der Schutzhülle 39 angeschlossen wird.

In den Figuren 3 und 4 ist eine Befestigungseinrichtung in Form einer hülsenförmigen Fassung 16 für die lichtdurchlässige Abdeckung 4 dargestellt. Diese Fassung wird mittels eines Klebers 19, welcher zwischen der Innenseite des Optiklumens 2 und der außen liegenden zylindrischen Mantelfläche 17 angeordnet ist, im distalen Endbereich des Optiklumens 2 befestigt. Bei dem Klebemittel 19 kann es sich um ein aushärtbares Klebemittel, beispielsweise auf Epoxydharzbasis handeln, welches durch eine Öffnung 21 in flüssiger Form eingebracht werden kann. Die Öffnung 21 erstreckt sich von der Außenseite der Kathetersonde 1 bis zur Mantelfläche 17 der Fassung 16. In der Mantelfläche 17 sind ferner, beispielsweise in Form von Eindrückungen, Vertiefungen 18 vorgesehen. Das eingebrachte Klebemittel füllt auch diese Vertiefungen 18 an. Es wird hierdurch eine Erhöhung der Haftfestigkeit der Abdeckung 16 an der Innenseite des Optiklumens 2 bei in axialer Richtung wirkenden Kräften erzielt.

Am dem distalen Ende der hülsenförmigen Fassung 16 ist ein nach innen zur Lumenachse hin gerichteter umlaufender Rand 20 vorgesehen, an welcher die Abdeckung 4 anliegt und nach außen hin abgestützt wird. In der Mantelfläche 17 sind die mehrere an separaten Stellen liegenden Vertiefungen 18 vorgesehen, wie es beispielsweise aus Figur 4 zu ersehen ist. Es ist auch möglich, umlaufende Vertiefungen in der Mantelfläche 17 vorzusehen. Die Vertiefungen können als Eindrückungen mit Materialverformung oder auch durch Materialendnahme an der Mantelfläche 17 hergestellt sein.

Zusammen mit der Abdeckung 4 oder in der Abdeckung 4 kann in der Fassung ein optisch wirksames Bauteil 22 oder können mehrere optisch wirksame Bauteile angeordnet sein. Die Fassung kann auch vom optisch wirksamen Bauteil 22 gebildet werden. Die Fassung kann optisch neutral ausgebildet sein, wobei am distalen Ende des Optikstranges 3 ein optisch wirksames Bauteil vorgesehen sein kann. Das optisch wirksame Bauteil kann eine oder mehrere optische Linsen aufweisen.

Als optisch wirksames Bauteil kann auch ein Videochip zum Einsatz kommen. Der Videochip wandelt das erfasste Bild in entsprechende Bildsignale, die über Anschlussdrähte, welche durch das Optiklumen 2 geführt sind, einer Auswerteeinrichtung am proximalem Kathetersondenende zugeführt werden. Der Videochip kann fest eingebaut sein und kann einen Bestandteil der Abdeckung 4 bilden. Bevorzugt wird der Videochip jedoch auswechselbar in das Optiklumen 2 eingeführt und gegen den als Bremse wirkenden Hülsenrand 20 angedrückt. Anstelle der galvanischen Übertragung der Signale des Videochips können dieses auch in Transpondertechnik telemetrisch übertragen werden. Der Optikstrang 3 benötigt dann nur noch Lichtleiterfasern zur Beleuchtung des zu beobachtenden Raums. Dieser Optikstrang kann auch in einem separatem Optiklumen angeordnet sein.

Durch entsprechende Bemessung der Längen für verschiedene Schutzhüllen 39 können unterschiedliche Längen der Kathetersonde 1 bei gleichbleibender Länge des Optikstranges 3 ausgeglichen werden. Beispielsweise reicht es aus, mit zwei Optikstranglängen von 150 cm und 300 cm Katheterlängen zwischen 20 cm und 185 cm mit Hilfe der unterschiedlichen Längen der Schutzhülle 39 abzudecken.

In Figur 5 ist ein Ende eines Optikdispensers 23 dargestellt, in welchem der Optikstrang 3 in steriler Anordnung in Bereitschaft halten werden kann. Das Ende des Optikdispensers 23 besitzt einen Anschluss 24 für eine Spüleinrichtung. Auf diese Weise ist es möglich den Optikstrang 3 im Dispenser 23 zu spülen und somit gegebenenfalls zu sterilisieren oder zu desinfizieren. Nach der Desinfektion kann der Optikdispenser 23 mit Wasser gespült und anschließend mit Druckluft getrocknet werden. Der Anschluss an die jeweils hierfür erforderlichen Geräte kann über den Anschluss 24 erfolgen.

### Bezugszeichenliste

- 1: Kathetersonde
- 2: Optiklumen
- 3: Optikstrang
- 4: Lichtdurchlässige Abdeckung
- 5: Optiklumenende
- 6: Optikstrangende
- 7: Längenausgleichseinrichtung
- 8: Krafteinleitungsstelle am proximalen Optikstrangende
- 9: Krafteinleitungsstelle am proximalen Optiklumenende
- 10: Kolbenzylinderanordnung
- 11: Zylinder
- 12: Kolben am proximalen Optiklumenende
- 13: Fixiereinrichtung (Klemmeinrichtung)
- 14: Feder (Druckfeder)
- 15: Kolben am proximalen Optikstrangende
- 16: Fassung
- 17: zylindrische Mantelfläche
- 18: Vertiefungen
- 19: Klebemittel
- 20: Hülsenrand
- 21: Öffnung
- 22: optischwirksames Bauteil
- 23: Optikdispenser
- 24: Anschluss für Spüleinrichtung
- 25: Führungsrohr
- 26: Kolbenstange
- 27: Handgriff
- 28: distales Sondenende
- 29 bis 31: Verbindungseinrichtungen
- 32: Adapter
- 33: Sondenansatzstück
- 34 bis 37: Lumenausgänge
- 38: Drehlager
- 39: Schutzhülle
- 40: Beleuchtungsanschluss
- 41: Okularanschluss
- 42: Kolbenstange

## Patentansprüche

1. Endoskop mit einer flexiblen und mehrere Lumen aufweisenden Katheter-sonde (1), wenigstens einem Optiklumen (2), in welchem ein Optikstrang (3) mit in Katheterlängsrichtung verlaufenden Lichtleitern vorgesehen ist, einer die distale Öffnung des Optiklumens ganz oder teilweise verschließenden lichtdurchlässigen Abdeckung (4) und einer zwischen dem praximalen Optiklumenende (5) und dem proximalen Optikstrangende (6) federnd wirkenden Längenausgleichsrichtung (7), mit welcher das distale Ende des Optikstranges (3) gegen die lichtdurchlässige Abdeckung (4) in Anlage gehalten wird,
**dadurch gekennzeichnet, dass** der Abstand einer starr mit dem proximalen Optikstrangende (6) verbundenen Krafteinleitungsstelle (8) der Längenausgleichseinrichtung (7) vom proximalen Optikstrangende (6) oder der Abstand einer starr mit dem proximalen Optiklumenende verbundenen Krafteinleitungsstelle (9) der Längenausgleichseinrichtung (7) vom proximalen Optiklumenende (5) einstellbar ist.

2. Endoskop nach Anspruch 1,
**dadurch gekennzeichnet, dass** zur Abstandseinstellung eine Kolbenzylinderanordnung (10), deren Zylinder (11) oder Kolben (12) mit dem proximalen Optiklumenende (5) und deren Kolben (12) oder Zylinder (11) mit dem proximalen Optikstrangende (6) starr verbunden sind, vorgesehen ist und dass der Kolben (12) im Zylinder (11) in unterschiedlichen axialen Positionen fixierbar ist.

3. Endoskop nach Anspruch 2,
**dadurch gekennzeichnet, dass** zur Fixierung der axialen Kolbenposition im Zylinder (11) eine Klemmeinrichtung (13) vorgesehen ist.

4. Endoskop nach einem der Ansprüche 1 bis 3,
**dadurch gekennzeichnet, dass** die abstandseinstellende Kolbenzylinderanordnung (10) und die Längenausgleichseinrichtung (7) in Reihe zwischen dem proximalen Optikstrangende (6) und dem proximalen Optiklumenende (5) angeordnet sind.

5. Endoskop nach einem der Ansprüche 1 bis 4,
**dadurch gekennzeichnet, dass** die Längenausgleichseinrichtung (7) und die abstandseinstellende Kolbenzylinderanordnung (10) in einem gemeinsamen Zylinder (11) angeordnet sind.

6. Endoskop nach einem der Ansprüche 1 bis 5,
**dadurch gekennzeichnet, dass** die Längenausgleichseinrichtung (7) eine Feder (14) aufweist, welche zwischen einem mit dem proximalen Optiklumenende (5) oder dem proximalen Optikstrangende (6) starr verbundenen Kolben (15) und dem mit dem proximalen Optikstrangende (6) oder dem proximalen Optiklumenende (5) starr verbundenen Zylinder (11) wirkt.

7. Endoskop nach Anspruch 6,
**dadurch gekennzeichnet, dass** die Feder (14) als Druckfeder ausgebildet ist.

8. Endoskop nach einem der Ansprüche 1 bis 7,
**dadurch gekennzeichnet, dass** der Optikstrang (3) im Zylinder (11) zumindest teilweise in einem vorzugsweise axial angeordneten Führungsrohr (25) geführt ist.

9. Endoskop nach einem der Ansprüche 1 bis 8,
**dadurch gekennzeichnet, dass** das Führungsrohr (25) mit dem proximalen Optikstrangende (6) oder dem proximalen Optiklumenende (5) fest verbunden ist.

10. Endoskop nach einem der Ansprüche 1 bis 9,
**dadurch gekennzeichnet, dass** die Längenausgleichseinrichtung im gemeinsamen Zylinder (11) zwei Kolben aufweist, von denen der eine Kolben (12) zur Abstandseinstellung und der andere Kolben (15) mit der Kraft der Druckfeder (14) beaufschlagt ist, und dass der Optikstrang (3) durch die beiden Kolben (12) und (15) in axialer Richtung geführt ist.

11. Endoskop nach einem der Ansprüche 1 bis 10,
**dadurch gekennzeichnet, dass** die lichtdurchlässige Abdeckung (4) in einer in das distale Optiklumenende (15) eingesetzten Fassung (16), welche mit einer zylindrischen Mantelfläche (17) an der Innenseite des distalen Optiklumenendes (15) befestigt ist, gehalten ist, wobei in der Mantelfläche (17) eine oder mehrere Vertiefungen (18) vorgesehen sind, welche mit einem Klebemittel (19) gefüllt ist oder sind.

12. Endoskop nach Anspruch 11,
**dadurch gekennzeichnet, dass** die Vertiefungen (18) an mehreren separaten Stellen auf der Mantelfläche (17) angeordnet sind.

13. Endoskop nach Anspruch 11 oder 12,
**dadurch gekennzeichnet, dass** die Vertiefungen (18) als Eindrückungen ausgebildet sind.

14. Endoskop nach einem der Ansprüche 11 bis 13,
**dadurch gekennzeichnet, dass** die Fassung (16) hülsenförmig ausgebildet ist und am distalen Ende einen zur Hülsenachse hin gerichteten einwärts gebogenen Hülsenrand (20) aufweist.

15. Endoskop nach einem der Ansprüche 11 bis 14,
**dadurch gekennzeichnet, dass** die Fassung (16) aus Metall, insbesondere Edelstahl besteht.

16. Endoskop nach einem der Ansprüche 11 bis 15,
**dadurch gekennzeichnet, dass** im Bereich der axialen Ausdehnung der Fassung (16) im Material des Optiklumens (2) eine von außen bis zur Mantelfläche (17) der Fassung (16) ragende Öffnung (21) zum Einfüllen des Klebemittels (19) vorgesehen ist.

17. Endoskop nach einem der Ansprüche 11 bis 16,
**dadurch gekennzeichnet, dass** in der Fassung (16) wenigstens ein optisch wirkendes Bauteil (22) fest oder auswechselbar angeordnet ist.

18. Endoskop nach Anspruch 17,
**dadurch gekennzeichnet, dass** das optisch wirksame Bauteil (22) die Abdeckung (4) bildet.

19. Endoskop nach Anspruch 17 oder 18,
**dadurch gekennzeichnet, dass** das optisch wirksame Bauteil (22) am distalen Ende des Optikstranges (3) vorgesehen, insbesondere befestigt ist.

20. Endoskop nach einem der Ansprüche 17 bis 19,
**dadurch gekennzeichnet, dass** das optisch wirksame Bauteil (22) eine oder mehrere optische Linsen aufweist.

21. Endoskop nach einem der Ansprüche 17 bis 20,
**dadurch gekennzeichnet, dass** das optisch wirksame Bauteil (22) als Videochip ausgebildet ist.

22. Endoskop nach einem der Ansprüche 1 bis 21,
**dadurch gekennzeichnet, dass** der Optikstrang (3) geeignet ist, eine Strahlung, insbesondere Licht mit einer Wellenlänge zu leiten, durch welche Autofluoreszenz von zu untersuchenden Körperzellen oder Körpergewebe angeregt wird.

23. Endoskop nach einem der Ansprüche 1 bis 22,
**dadurch gekennzeichnet, dass** für Kathetersonden mit unterschiedlichen Längen ein Optikstrang (3) mit einheitlicher Länge vorgesehen ist, wobei zwischen dem proximalen Ende der Kathetersonde (1) und der Längenausgleichseinrichtung (7) jeweilige die unterschiedlichen Längen der Kathetersonden (1) ausgleichende Schutzhüllen (39) mit unterschiedlichen Längen, durch welche über das proximale Ende der Kathetersonde (1) überstehende Teil des Optikstranges (3) geführt ist, anzuordnen sind.

24. Endoskop nach einem der Ansprüche 1 bis 23,
**dadurch gekennzeichnet, dass** zur Bereitstellung des Optikstranges (3) außerhalb der Kathetersonde (1) ein schlauchförmiger Optikdispenser (23) vorgesehen ist, an dessen einem Ende ein Anschluss (24) für eine Spüleinrichtung vorgesehen ist.

25. Endoskop nach einem der Ansprüche 1 bis 24,
**dadurch gekennzeichnet, dass** die Kathetersonde (1) ein an seinem proximalen Ende betätigbares Steuerelement aufweist, welches am distalen Sondenende (28) oder in der Nähe davon zum Abbiegen des Sondenendes befestigt ist und in axialer Richtung an der Sonde, insbesondere in einem Steuerlumen der Kathetersonde (1) beweglich geführt ist.

## Claims

1. An endoscope comprising a flexible catheter probe (1) having a plurality of lumens, at least one optical lumen (2) in which there is provided an optical strand (3) with optical fibres extending in the longitudinal direction of the catheter, a transparent cover (4) which entirely or partially closes the distal opening of the optical lumen and a length compensating device (7) which acts resiliently between the proximal end (5) of the optical lumen and the proximal end (6) of the optical strand and with which the distal end of the optical strand (3) is held in contact against the transparent cover (4),
**characterised in that** the spacing of a force application location (8) of the length compensating device (7), which location is rigidly connected to the proximal end (6) of the optical strand, from the proximal end (6) of the optical strand or the spacing of a force application location (9) of the length compensating device (7), which location (9) is rigidly connected to the proximal end (5) of the optical lumen, from the proximal end (5) of the optical lumen, is adjustable.

2. An endoscope according to claim 1
**characterised in that** for spacing adjustment there is provided a piston-cylinder arrangement (10) whose cylinder (11) or piston (12) is rigidly connected to the proximal end (5) of the optical lumen and whose piston (12) or cylinder (11) is rigidly connected to the proximal end (6) of the optical strand and that the piston (12) can be fixed in the cylinder (11) in different axial positions.

3. An endoscope according to claim 2
**characterised in that** a clamping device (13) is provided for fixing the axial position of the piston in the cylinder (11).

4. An endoscope according to one of claims 1 to 3
**characterised in that** the spacing-adjusting piston-cylinder arrangement (10) and the length compensating device (7) are arranged in succession between the proximal end (6) of the optical strand and the proximal end (5) of the optical lumen.

5. An endoscope according to one of claims 1 to 4
**characterised in that** the length compensating device (7) and the spacing-adjusting piston-cylinder arrangement (10) are arranged in a common cylinder (11).

6. An endoscope according to one of claims 1 to 5
**characterised in that** the length compensating device (7) has a spring (14) which is operative between a piston (15) rigidly connected to the proximal end (5) of the optical lumen or the proximal end (6) of the optical strand, and the cylinder (11) which is rigidly connected to the proximal end (6) of the optical strand or the proximal end (5) of the optical lumen.

7. An endoscope according to claim 6
**characterised in that** the spring (14) is in the form of a compression spring.

8. An endoscope according to one of claims 1 to 7
**characterised in that** the optical strand (3) is guided in the cylinder (11) at least partially in a preferably axially arranged guide tube (25).

9. An endoscope according to one of claims 1 to 8
**characterised in that** the guide tube (25) is fixedly connected to the proximal end (6) of the optical strand or the proximal end (5) of the optical lumen.

10. An endoscope according to one of claims 1 to 9
**characterised in that** the length compensating device has in the common cylinder (11) two pistons of which the one piston (12) is for spacing adjustment and the other piston (15) is acted upon by the force of the compression spring (14) and that the optical strand (3) is guided in the axial direction through the two pistons (12) and (15).

11. An endoscope according to one of claims 1 to 10,
**characterised in that** the transparent cover (4) is held in a holder (16) which is fitted into the distal end (5) of the optical lumen and which is fixed with a cylindrical peripheral surface (17) to the inside of the distal end (5) of the optical lumen, wherein provided in the peripheral surface (17) are one or more recesses (18) which is or are filled with an adhesive agent (19).

12. An endoscope according to claim 11
**characterised in that** the recesses (18) are arranged at a plurality of separate locations on the peripheral surface (17).

13. An endoscope according to claim 11 or claim 12
**characterised in that** the recesses (18) are in the form of impressions.

14. An endoscope according to one of claims 11 to 13
**characterised in that** the holder (16) is of a sleeve-shaped configuration and at the distal end has a sleeve edge (20) which is bent inwardly and which is directed towards the axis of the sleeve.

15. An endoscope according to one of claims 11 to 14
**characterised in that** the holder (16) comprises metal, in particular highquality steel.

16. An endoscope according to one of claims 11 to 15
**characterised in that** provided in the region of the axial extent of the holder (16) in the material of the optical lumen (2) is an opening (21) which extends from the exterior as far as the peripheral surface (17) of the holder (16), for introducing the adhesive agent (19).

17. An endoscope according to one of claims 11 to 16
**characterised in that** at least one optically operative component (22) is fixedly or interchangeably arranged in the holder (16).

18. An endoscope according to claim 17
**characterised in that** the optically operative component (22) forms the cover (4).

19. An endoscope according to claim 17 or claim 18
**characterised in that** the optically operative component (22) is provided and in particular fixed at the distal end of the optical strand (3).

20. An endoscope according to one of claims 17 to 19
**characterised in that** the optically operative component (22) has one or more optical lenses.

21. An endoscope according to one of claims 17 to 20
**characterised in that** the optically operative component (22) is in the form of a video chip.

22. An endoscope according to one of claims 1 to 21
**characterised in that** the optical strand (3) is suitable for passing a radiation, in particular light at a wavelength by which autofluorescence of body tissue or body cells to be investigated is stimulated.

23. An endoscope according to one of claims 1 to 22
**characterised in that** an optical strand (3) of uniform length is provided for catheter probes of different lengths, wherein there are to be arranged between the proximal end of the catheter probe (1) and the length compensating device (7), respective protective casings (39) of differing lengths, which compensate for the differing lengths of the catheter probes (1) and through which is guided the part of the optical strand (3), which projects beyond the proximal end of the catheter probe (1).

24. An endoscope according to one of claims 1 to 23
**characterised in that** a tubular optical dispenser (23) is provided outside the catheter probe (1) for supplying the optical strand (3), a connection (24) for a flushing device being provided at one end of the optical dispenser.

25. An endoscope according to one of claims 1 to 24
**characterised in that** the catheter probe (1) has a control element which is actuable at its proximal end and which is fixed at the distal end (28) of the probe or in the proximity thereof for bending the end of the probe and is guided movably in the axial direction on the probe, in particular in a control lumen of the catheter probe (1).

## Revendications

1. Endoscope comprenant un cathéter (1) flexible qui présente plusieurs lumens, au moins un lumen optique (2), dans lequel est prévu un cordon optique (3) avec des guides de lumière s'étendant dans la direction longitudinale du cathéter, un recouvrement (4) transparent qui ferme en tout ou partie l'ouverture distale du lumen optique, et un dispositif de compensation de longueur (7), qui agit élastiquement entre l'extrémité proximale (5) du lumen optique et l'extrémité proximale (6) du cordon optique et par lequel l'extrémité distale du cordon optique (3) est maintenue au contact du recouvrement transparent (4), **caractérisé en ce que** la distance entre un point de transmission des forces (8), raccordé rigidement à l'extrémité proximale (6) du cordon optique, du dispositif de compensation de longueur (7) et l'extrémité proximale (6) du cordon optique ou la distance entre un point de transmission de forces (9), raccordé rigidement à l'extrémité proximale (5) du lumen optique, du dispositif de compensation de longueur (7) et l'extrémité proximale (5) du lumen optique est réglable.

2. Endoscope suivant la revendication 1, **caractérisé en ce qu'**il est prévu, pour le réglage de distance, un agencement de cylindre - piston (10), dont le cylindre (11) ou le piston (12) est raccordé rigidement à l'extrémité proximale (5) du lumen optique et dont le piston (12) ou le cylindre (11) est raccordé rigidement à l'extrémité proximale (6) du cordon optique, et que le piston (12) peut être fixé dans le cylindre (11) dans des positions axiales différentes.

3. Endoscope suivant la revendication 2, **caractérisé en ce qu'**un dispositif de serrage (13) est prévu pour fixer la position axiale du piston dans le cylindre (11).

4. Endoscope suivant l'une des revendications 1 à 3, **caractérisé en ce que** l'agencement de cylindre - piston (10) réglant la distance et le dispositif de compensation de longueur (7) sont disposés en série entre l'extrémité proximale (6) du cordon optique et l'extrémité proximale (5) du lumen optique.

5. Endoscope suivant l'une des revendications 1 à 4, **caractérisé en ce que** le dispositif de compensation de longueur (7) et l'agencement de cylindre - piston (10), réglant la distance, sont disposés dans un cylindre commun (11).

6. Endoscope suivant l'une des revendications 1 à 5, **caractérisé en ce que** le dispositif de compensation de longueur (7) présente un ressort (14), qui agit entre un piston (15), raccordé rigidement à l'extrémité proximale (5) du lumen optique ou à l'extrémité proximale (6) du cordon optique, et le cylindre (11), raccordé rigidement à l'extrémité proximale (6) du cordon optique ou à l'-extrémité proximale (5) du lumen optique.

7. Endoscope suivant la revendication 6, **caractérisé en ce que** le ressort (14) est réalisé sous forme de ressort de pression.

8. Endoscope suivant l'une des revendications 1 à 7, **caractérisé en ce que** le cordon optique (3) est guidé dans le cylindre (11) au moins en partie dans un tube de guidage (25), disposé de préférence dans la direction axiale.

9. Endoscope suivant l'une des revendications 1 à 8, **caractérisé en ce que** le tube de guidage (25) est raccordé fixement à l'extrémité proximale (6) du cordon optique ou à l'extrémité proximale (5) du lumen optique.

10. Endoscope suivant l'une des revendications 1 à 9, **caractérisé en ce que** le dispositif de compensation de longueur présente dans le cylindre commun (11) deux pistons, dont l'un (12) est sollicité pour le réglage de distance et l'autre piston (15) est sollicité par la force du ressort de pression (14), et que le cordon optique (3) est guidé dans la direction axiale par les deux pistons (12) et (15).

11. Endoscope suivant l'une des revendications 1 à 10, **caractérisé en ce que** le recouvrement transparent (4) est maintenu dans une monture (16) mise en place dans l'extrémité distale (15) du lumen optique, laquelle monture est fixée par une surface d'enveloppe cylindrique (17) sur le côté intérieur de l'extrémité distale (15) du lumen optique, une ou plusieurs cavités (18), qui sont remplies d'un agent adhésif (19) ou sont un adhésif, étant alors prévues dans la surface d'enveloppe (17).

12. Endoscope suivant la revendication 11, **caractérisé en ce que** les cavités (18) sont disposées sur la surface d'enveloppe (17) en plusieurs endroits séparés.

13. Endoscope suivant l'une des revendications 11 et 12, **caractérisé en ce que** les cavités (18) sont réalisées sous forme d'empreintes.

14. Endoscope suivant l'une des revendications 11 à 13, **caractérisé en ce que** la monture (16) est configurée en forme de douille et présente à l'extrémité distale un bord de douille (20) plié en dedans en direction de l'axe de la douille.

15. Endoscope suivant l'une des revendications 11 à 14, **caractérisé en ce que** la monture (16) se compose de métal, en particulier d'acier spécial.

16. Endoscope suivant l'une des revendications 11 à 15, **caractérisé en ce qu'**il est prévu, dans la zone de l'extension axiale de la monture (16) dans le matériau du lumen optique (2), une ouverture (21) pénétrant de l'extérieur jusqu'à la surface d'enveloppe (17) de la monture (16) pour le remplissage d'agent adhésif (19).

17. Endoscope suivant l'une des revendications 11 à 15, **caractérisé en ce qu'**au moins un composant optique (22) est disposé fixement ou de manière interchangeable dans la monture (16).

18. Endoscope suivant la revendication 17, **caractérisé en ce que** le composant optique (22) forme le recouvrement (4).

19. Endoscope suivant l'une des revendications 17 et 18, **caractérisé en ce que** le composant optique (22) est prévu à l'extrémité distale du cordon optique (3), en particulier fixé.

20. Endoscope suivant l'une des revendications 17 à 19, **caractérisé en ce que** le composant optique (22) présente une ou plusieurs lentilles optiques.

21. Endoscope suivant l'une des revendications 17 à 20, **caractérisé en ce que** le composant optique (22) est réalisé sous forme de puce vidéo.

22. Endoscope suivant l'une des revendications 1 à 21, **caractérisé en ce que** le cordon optique (3) est adapté à transmettre un rayonnement, en particulier de la lumière d'une longueur d'onde, par laquelle est excitée de l'autofluorescence de cellules corporelles ou de tissus corporels à analyser.

23. Endoscope suivant l'une des revendications 1 à 22, **caractérisé en ce qu'**il est prévu un cordon optique (3) de longueur uniforme pour des cathéters de longueurs différentes, des gaines de protection (39) respectives de longueurs différentes, qui compensent les longueurs différentes des cathéters (1), pouvant être alors agencées entre l'extrémité proximale du cathéter (1) et le dispositif de compensation de longueur (7), gaines au travers desquelles est guidée la partie du cordon optique (3) dépassant de l'extrémité proximale du cathéter (1).

24. Endoscope suivant l'une des revendications 1 à 23, **caractérisé en ce qu'**il est prévu un distributeur optique (23) en forme de tuyau flexible pour tenir prêt le cordon optique (3) à l'extérieur du cathéter (1), distributeur sur une extrémité duquel est prévu un raccord (24) pour un dispositif de lavage.

25. Endoscope suivant l'une des revendications 1 à 24, **caractérisé en ce que** le cathéter (1) présente un élément de commande actionnable sur son extrémité proximale, lequel élément est fixé sur l'extrémité distale (28) du cathéter ou au voisinage de cette dernière pour plier l'extrémité du cathéter et est guidé de façon mobile, dans la direction axiale, sur le cathéter (1), en particulier dans un lumen de commande de ce dernier.
